(19) Europäisches Patentamt
European Patent Office
Office européen des brevets

(11) Veröffentlichungsnummer : **0 341 538 B1**

(12) # EUROPÄISCHE PATENTSCHRIFT

(45) Veröffentlichungstag der Patentschrift :
**18.03.92 Patentblatt 92/12**

(51) Int. Cl.$^5$ : **C07C 43/15,** A61K 7/46

(21) Anmeldenummer : **89107878.4**

(22) Anmeldetag : **29.04.89**

(54) **1-Tert.-Butoxy-omega-alkene und deren Verwendung als Riechstoffe.**

(30) Priorität : **04.05.88 DE 3815044**

(43) Veröffentlichungstag der Anmeldung :
**15.11.89 Patentblatt 89/46**

(45) Bekanntmachung des Hinweises auf die Patenterteilung :
**18.03.92 Patentblatt 92/12**

(84) Benannte Vertragsstaaten :
**CH DE FR GB IT LI**

(56) Entgegenhaltungen :
**DE-A- 3 712 075**
**DE-B- 1 125 910**
**GB-A- 2 085 881**
**US-A- 2 667 517**

(73) Patentinhaber : **BASF Aktiengesellschaft**
**Carl-Bosch-Strasse 38**
**W-6700 Ludwigshafen (DE)**

(72) Erfinder : **Becker, Rainer, Dr.**
**Im Haseneck 22**
**W-6702 Bad Duerkheim (DE)**
Erfinder : **Eckhardt, Heinz, Dr.**
**Ruedigerstrasse 7**
**W-6700 Ludwigshafen (DE)**
Erfinder : **Eggersdorfer, Manfred, Dr.**
**Hannongstrasse 18**
**W-6710 Frankenthal (DE)**
Erfinder : **Schindler, Gerhard**
**Collinistrasse 5**
**W-6800 Mannheim (DE)**
Erfinder : **Jansen, Klaas**
**Tannenstrasse 30**
**W-6700 Ludwigshafen (DE)**

EP 0 341 538 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 1-tert.-Butoxy-ω-alkene der allgemeinen Formel I

$$CH_2{=}CH{-}(CH_2)_n{-}O{-}C(CH_3)_3 \qquad (I),$$

in der n für eine ganze Zahl von 4 bis 10 steht sowie deren Verwendung als Riechstoffe, d.h. zur Verleihung, Verbesserung oder Modifizierung der Dufteigenschaften von Parfüms oder zu parfümerierenden Produkten und 1-tert.-Butoxy-ω-alkene der Formel I enthaltende Riechstoffkompositionen.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an Geruchsverbesserern für Produkte des täglichen Bedarfs, wie Reinigungsmitteln, Kosmetika, Leimen etc. hat die Riechstoffindustrie ständig Bedarf an neuen Riechstoffen, die allein oder in Form von Kompositionen wertvolle Parfüms bzw. Duftstoffe mit interessanten Duftnoten darstellen. Da aufgrund wenig bekannter Zusammenhänge zwischen Struktur und Riechstoffeigenschaften eine gezielte Synthese von Riechstoffen mit gewünschten olfaktorischen Eigenschaften nicht möglich ist, besteht die Aufgabe, solche Verbindungen aufzufinden, die wertvolle Riechstoffqualitäten besitzen.

Es war daher die Aufgabe der Erfindung, neue interessante Riechstoffe zu entwickeln, die auf möglichst einfache Weise aus gut zugänglichen und daher billigen Ausgangsstoffen hergestellt werden können.

ω-Alkenole wie ω-Nonenol (Arctander Nr. 2357; vgl. S. Arctander, "Perfume and Flavor Chemicals", Mont Clair, New York, 1969) oder ω-Decenol (Arctander Nr. 844) weisen mehr oder weniger ausgeprägte blumige Duftnoten auf, die im Falle des Decenols an Rose erinnern. ω-Decenylacetat (Arctander Nr. 846) und ω-Decenylpropionat (Arctander Nr. 847) weisen ebenfalls interessante blumige Duftnoten auf, zeigen aber den erheblichen Nachteil, daß sie erheblich instabil gegen Alkalien sind. ω-Hexenylmethylether (Arctander Nr. 2054) ist zwar alkalistabil, zeigt dafür aber den Nachteil der sehr raschen Flüchtigkeit und ist außerdem nach Angaben in loc. cit nur sehr schlecht zugänglich.

Es wurde nun überraschenderweise gefunden, daß die neuen 1-tert.-Butoxy-ω-alkene der Formel I nicht nur vorteilhafte Riechstoffeigenschaften aufweisen, sondern auch gut haftend und alkalistabil sind.

So weist beispielsweise das 1-tert.-Butoxy-hex-5-en einen sehr intensiven grünen und krautigen Geruch mit einer blumigen, an Tagetes erinnernden Note auf, die selbst beiniedriger Dosierung in Parfümkompositionen deutlich in Erscheinung tritt. Gerade dieser besonders blumige Effekt macht die Verbindung zu einem interessanten Riechstoff. Wegen seiner Flüchtigkeit ist er zur Modifizierung der Kopfnote von Parfümkompositionen geeignet. Besonders wirkungsvoll zeigt sich diese Verbindung in frischen krautigen Parfümtypen, wobei Dosierungen von 6 bis 8 Gew.% möglich sind. In blumigen Kompositionen, wie Gartennelke und Geißblatt, aber auch Maiglöckchen und Rose zeigt bereits eine Menge von 1 bis 3 % eine deutliche Wirkung.

Darüber hinaus hat beispielsweise das 1-tert.-Butoxy-hex-5-en Bedeutung als Zwischenprodukt für ein technisch einfach durchführbares Verfahren zur Herstellung von E-7,Z-9-Dodecadienylacetat, dem Pheromon des bekreuzten Traubenwicklers, Lobesia Botrana, welches zur Kontrolle dieser Insekten mittels der Konfusionsmethode von Interesse ist. Dieses Pheromon wurde 1973 erstmals beschrieben (vgl. US 3 845 108), die bislang bekannten Herstellungsverfahren sind jedoch technisch sehr umständlich.

Demgegenüber eröffnet sich ein technisch relativ einfach durchführbarer Weg ausgehend von dem erfindungsgemäßen 1-tert.-Butoxy-hex-5-en. Hierzu wird dieses in Gegenwart von Rhodium-Triphenylphosphinkomplexen in 7-tert.-Butoxy-heptanal überführt, aus welchem durch basenkatalysierte Umsetzung mit Acetylen in einem aprotischen organischen Lösungsmittel 3-Hydroxy-9-tert.-butoxy-1-nonin erhalten werden kann. Letzteres gibt durch Meyer-Schuster-Umlagerung (vgl. Houben-Weyl, Methoden der Organischen Chemie, Bd. VII/2 (1973), Seiten 907-927) 9-tert.-Butoxy-2-nonen-1-al, dessen Verwendung zur Synthese des gesuchten Pheromonwirkstoffs problemlos und in GB-A- 2 098 609 bzw. Liebigs Ann. Chem. Nr. 2 (1981), Seiten 1705-20 beschrieben ist.

Gegenstand der Erfindung ist weiterhin ein Verfahren zur Herstellung der neuen 1-tert.-Butoxy-ω-alkene, das dadurch gekennzeichnet ist, daß man die entsprechenden ω-Alkenole der allgemeinen Formel II

$$CH_2{=}CH{-}(CH_2)_n{-}OH \qquad (II),$$

in Gegenwart von sauren Katalysatoren mit Isobuten umsetzt.

Die ω-Alkenole der Formel II sind bekannte Verbindungen, die beispielsweise auf einfache Weise durch

partielles Dehydratisieren von α,ω-Diolen in hoher Selektivität und Reinheit hergestellt werden können (vgl. DE 35 10 568). Bevorzugte Alkenole der Formel II sind Hexenol, Octenol, Nonenol, Decenol und Dodecenol.

Die Reaktionstemperatur für die tert.-Butylierung kann 0 bis 100°C, vorzugsweise 20 bis 50°C betragen.

Die Umsetzung kann sowohl lösungsmittelfrei als auch in einem inerten protonenfreien organischen Lösungsmittel durchgeführt werden. Als geeignete Lösungsmittel seien genannt: Kohlenwasserstoffe, wie Hexan, Heptan, Octan, Cyclohexan oder deren Gemische; Ether, wie Methyl-tert.-butylether oder Tetrahydrofuran.

Als saure Katalysatoren kommen anorganische Säuren, wie Schwefelsäure, Phosphorsäure oder Salzsäure sowie organische Säuren, wie Essigsäure, Oxalsäure, p-Toluolsulfonsäure oder aber saure Ionenaustauscher in Betracht. Man kann bei Normaldruck oder mit Drucken von 1 bis 10 bar arbeiten.

Da es sich bei der tert.-Butylierung um ein an sich bekanntes Verfahren handelt, erübrigen sich nähere Angaben.

Die erfindungsgemäßen 1-tert.-Butoxy-ω-alkene sind interessante Riechstoffe und wertvolle Zwischenprodukte für einen neuen vorteilhaften Syntheseweg für E-7,Z-9-Dodecadienylacetat, dem Pheromon des bekreuzten Traubenwicklers, Lobesia Botrana. Sie können auf einfache Weise aus gut zugänglichen Ausgangsstoffen hergestellt werden.

Beispiel 1

200 g Hex-5-en-1-ol wurden in 250 ml Methyl-tert.-butylether gelöst und mit 100 g saurem Kationenaustauscher (SPC 118 der Firma Bayer in der $H^+$-Form) versetzt. Anschließend wurde in die erhaltene Suspension bis zur Sättigung Isobutylen eingeleitet und der Fortgang der Reaktion mittels dünnschichtchromatographischer Untersuchung verfolgt. Nach 4 Stunden (h) war alles Hex-5-en-1-ol umgesetzt. Durch fraktionierte Destillation des Reaktionsansatzes wurden 234 g 1-tert.-Butoxy-5-en vom Siedepunkt $Kp_{32\ mbar}$ = 66-67°C erhalten. Die Reinheit des Produktes betrug gemäß gaschromatographischer Bestimmung 98,5 %; die Ausbeute 75 % der Theorie.
Duftnote: sehr intensiv grün und krautig mit einer blumigen, an Tagetes erinnernden Note.

Beispiele 2-3

Analog Beispiel 1 wurden durch Umsetzen von jeweils 2 Mol Alkenol in Methyl-tert.-butylether in Gegenwart von 100 g des Ionenaustauschers SPC 118 mit Isobutylen die aus der folgenden Tabelle ersichtlichen Alkenyl-tert.-butylether erhalten.

Tabelle

| Beispiel | Alkenyl-tert.-butylether | n | Kp [°C/mbar] | Ausbeute [% der Theorie] | Duftnote |
|---|---|---|---|---|---|
| 2 | Oct-7-en-1-ol-tert.-butylether | 6 | 53-55/0,6 | 75 % | grün, krautig blumig (Tagetes) |
| 3 | Nona-8-en-1-ol-tert.-butylether | 7 | 110-112/20 | 82 % | trocken pudrig (Tagetes, Salbei) |

Anwendungsbeispiel

Unter Verwendung von 1-tert.-Butoxy-hex-5-en wurde ein Cremeparfüm folgender Zusammensetzung hergestellt:

| | | |
|---|---|---|
| Benzylisoeugenol | 50 | Gew.-Teile |
| Dimethylbenzylcarbinylacetat | 50 | " |
| Tetrahydrolinalool | 150 | " |
| Tetrahydrolinalylacetat | 200 | " |
| Citronellylacetat | 50 | " |
| Phenylacetaldehyddimethylacetal | 40 | " |
| Balinol® (Firma FDO) | 20 | " |
| Phenylethylalkohol | 90 | " |
| Hedione® (Firma Firmenich) | 60 | " |
| Lysmeral® (BASF) | 60 | " |
| Anisaldehyd | 30 | " |
| Geraniol | 80 | " |
| Cedrylacetat | 60 | " |
| Dipropylenglycol | 20 | " |
| 1-tert.-Butoxy-hex-5-en | 40 | |
| | 1000 | Gew.-Teile |

Der Zusatz von 1-tert.-Butoxy-hex-5-en gibt der Komposition eine angenehm frische, etwas fruchtige Note und den typischen Eindruck von Tagetes. Außerdem erhält die Komposition mehr Ausstralung.

## Patentansprüche

1. 1-tert.-Butoxy-ω-alkene der allgemeinen Formel I

$$CH_2{=}CH{-}(CH_2)_n{-}O{-}C(CH_3)_3 \qquad (I),$$

in der n für eine ganze Zahl von 4 bis 10 steht.

2. Verwendung von 1-tert.-Butoxy-ω-alkenen der allgemeinen Formel I gemäß Anspruch 1 zur Verleihung, Verbesserung oder Modifizierung der Dufteigenschaften von Parfüms oder parfümierten Produkten.

3. Riechstoffkompositionen, enthaltend 1-tert.-Butoxy-ω-alkene der allgemeinen Formel I gemäß Anspruch 1.

4. Verfahren zur Herstellung der 1-tert.-Butoxy-ω-alkene der allgemeinen Formel I gemäß Anspruch 1, dadurch gekennzeichnet, daß man die entsprechenden Alkenole der allgemeinen Formel II

$$CH_2{=}CH{-}(CH_2)_n{-}OH \qquad (II)$$

in Gegenwart von sauren Katalysatoren mit Isobutylen umsetzt.

## Revendications

1. 1-tert.-Butoxy-ω-alcènes de formule générale I

$$CH_2{=}CH{-}(-CH_2-)_n{-}o{-}C(CH_3)_3 \qquad (I),$$

dans laquelle n est mis pour un nombre entier de 4 à 10

2. Utilisation des 1-tert.-butoxy-ω-alcènes de formule générale I selon la revendication 1 pour donner, améliorer ou modifier des caractéristiques odorantes de parfums ou de produits parfumés.

3. Compositions parfumées contenant des 1-tert.-butoxy-ω-alcènes de formule générale I selon la revendication 1.

4. Procédé de préparation des 1-tert.-butoxy-ω-alcènes de formule générale I selon la revendication 1,

caractérisé en ce qu'on fait réagir avec de l'isobutylène on présence de catalyseurs acides les alcénols correspondants de formule générale II

$$CH_2=CH-(CH_2)_n-OH \quad (II).$$

## Claims

1. A 1-tert-butoxy-ω-alkene of the formula I

$$CH_2=CH-(CH_2)_n-O-C(CH_3)_3 \quad (I)$$

where n is an integer from 4 to 10.

2. The use of a 1-tert-butoxy-ω-alkene of the formula I as claimed in claim 1 for imparting fragrance properties to perfumes or to perfumed products, or for improving or modifying the fragrance properties of the said perfumes or products.

3. A scent composition containing a 1-tert-butoxy-ω-alkene of the formula I as claimed in claim 1.

4. A process for the preparation of a 1-tert-butoxy-ω-alkene of the formula I as claimed in claim 1, wherein the corresponding alkenol of the formula II

$$CH_2=CH-(CH_2)_n-OH \quad (II)$$

is reacted with isobutene in the presence of an acidic catalyst.